(19) Europäisches Patentamt
European Patent Office
Office européen des brevets

(11) Veröffentlichungsnummer: **0 037 973**
**B1**

(12) # EUROPÄISCHE PATENTSCHRIFT

(45) Veröffentlichungstag der Patentschrift: **30.05.84**

(21) Anmeldenummer: **81102486.8**

(22) Anmeldetag: **02.04.81**

(51) Int. Cl.³: **C 07 J 1/00,** C 07 C 47/575, C 07 C 49/647, C 07 C 49/753, C 07 F 7/08 // C07C69/157

(54) **Verfahren zur Synthese von Östron bzw. Östronderivaten.**

(30) Priorität: **12.04.80 DE 3014120**

(43) Veröffentlichungstag der Anmeldung: **21.10.81 Patentblatt 81/42**

(45) Bekanntmachung des Hinweises auf die Patenterteilung: **30.05.84 Patentblatt 84/22**

(84) Benannte Vertragsstaaten: **DE FR GB**

(56) Entgegenhaltungen:
**DE - A - 2 523 179**

**CHEMICAL ABSTRACTS, Band 75, Nr. 11, 13. September 1971, Seite 492, Nr. 77122x Columbus, Ohio, U.S.A. G.S. GRINENKO et al.: "Total synthesis of dl-alkyl-1,3,5(10)-gonatrien-3-ol-17-one methyl ethers"**
**JOURNAL OF THE AMERICAN CHEMICAL SOCIETY, Band 99, Nr. 10, 11. Mai 1977, Seiten 3461-3466 Washington D.C., U.S.A. TETSUJI KAMETANI et al.: "A stereoselective total synthesis of estrone by an intramolecular cycloaddition reaction of olefinic o-quinodimethane"**

(73) Patentinhaber: **HOECHST AKTIENGESELLSCHAFT, Postfach 80 03 20, D-6230 Frankfurt am Main 80 (DE)**

(72) Erfinder: **Quinkert, Gerhard, Prof. Dr., Schauinsland 32, D-6241 Glashütten/Taunus (DE)**
Erfinder: **Weber, Wolf-Dietrich, Dr., Sindlinger Bahnstrasse 58, D-6230 Frankfurt am Main 80 (DE)**
Erfinder: **Schwartz, Ulrich, Hügelstrasse 214, D-6000 Frankfurt am Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

1. Die Erfindung betrifft ein Verfahren zur Synthese von Östron bzw. Östronderivaten, die gegebenenfalls eine 10-α-Hydroxygruppe oder eine 9(11)-Doppelbindung enthalten.

Derivate und chemische Varianten des steroidalen Follikelhormons (Östrogen) spielen als potente Wirkstoffe im Sexualzyklus der Frau oder als wichtige Komponente der oralen hormonalen Kontrazeption eine relevante Rolle im praxisorientierten Anwendungsbereich der Gynäkologie. Darüber hinaus stellen die genannten Östrogene wichtige (chemische) Ausgangs- oder Zwischenprodukte zur chemischen Umwandlung in 19-Norsteroide (Gonan-Abkömmlinge) dar. Letztere besitzen in Form ihrer vielfältigen chemischen und strukturellen Varianten ebenfalls grosses praktisches Interesse zur Behandlung von diversen Sexualfunktionsstörungen oder als weitere Komponente von oralen hormonalen Kontrazeptiva.

Die industrielle Herstellung der Östrogene erfolgte in früheren Jahren hauptsächlich aus dem pflanzlichen Steroidsaponin Diosgenin. Als Folge der weltweiten Verteuerung und Verknappung des pflanzlichen Ausgangsmaterials fand in letzter Zeit eine Verlagerung der industriellen Produktion in Richtung der Totalsynthese dieser Stoffklasse statt, die heute in der Regel gegenüber der Herstellungsweise aus Naturstoffen beträchtlich ökonomischer und zum Teil auch hinsichtlich der strukturellen Variationsbreite vielfältiger und attraktiver ist.

Aus der deutschen Offenlegungsschrift Nr. 2523179 ist ein Verfahren zur Herstellung von Gona-1,3,5(10),9(11)-tetraenen bekannt, nach welchem Gona-1,3,5(10)-triene unter Zusatz von Elektrolytsalzen in Gegenwart von Alkoholen, Wasser und Protonenakzeptoren elektrolysiert werden. Die sich bildenden 9-α- und 9-β-Alkoxy- bzw. Hydroxyverbindungen werden durch saure Behandlung in Gona-1,3,5(10),9(11)-tetraene umgewandelt. Das erfindungsgemässe Mehrstufenverfahren, insbesondere die darin wichtige Herstellung der 9-α-Hydroxyöstron-3-methyläther durch Bestrahlung von o-Chinodimethanderivaten mit UV-Licht bei >340 nm sind dort weder nahegelegt noch beschrieben.

Nach C.A. 75, Nr. 11, S. 492, 77122x (1971) werden ebenfalls substituierte Gonatetraene oder durch deren Hydrierung die entsprechenden Gonatriene erhalten. Auch bei diesem Verfahren, das von Tetrahydroindanderivaten ausgeht und nach einer Hydrierung und nachfolgender Wittigredaktion mit $MeOC_6H_4CH_2Cl$ zum substituierten Gona-1,3,5,6-tetraen führt, ist keinerlei Hinweis auf das erfindungsgemässe Vielstufenverfahren und die darin wesentliche UV-Bestrahlung gegeben, bei welchem die vier Ringelemente der Östrone in überraschender Weise aufgebaut und miteinander verknüpft werden.

Das 4teilige Steroidringsystem wird vereinbarungsgemäss in die Ringelemente A, B, C und D unterteilt. Bei der chemischen Totalsynthese werden aus Variationen von Untereinheiten Synthesewege beschritten, die in möglichst rationaler und einfacher Art unter Einbeziehung stereoselektiver und regiospezifischer Reaktionsführungen zu den gewünschten Zielverbindungen des Musters A, B, C, D führen.

Es wurde nun gefunden, dass ein totalsynthetischer Aufbau von Östron bzw. Östronderivaten nach folgendem Schema

A+D→AD→ABCD möglich ist.

Die Erfindung betrifft daher ein Verfahren zur Herstellung von Östron oder Östronderivaten, das dadurch gekennzeichnet ist, dass man den Aldehyd der Formel 6

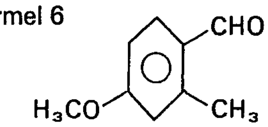

mit der Grignard-Verbindung aus 1-Bromvinyltrimethylsilan zur Verbindung der Formel 8

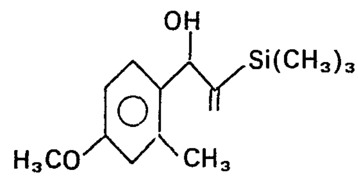

umsetzt, diese zur Verbindung der Formel 11

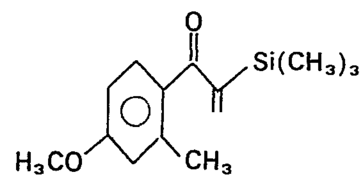

oxidiert (Ring-A-Baustein), diese durch mit Alkali- und Erdalkalialkoholaten katalysierte Michael-Reaktion mit der Verbindung der Formel 12 c

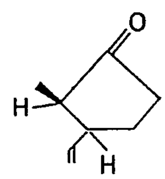

(Ring-D-Baustein), welche durch Reaktion von 2-Vinylcyclopropan-1,1-dicarbonsäuredimethylester der Formel 7

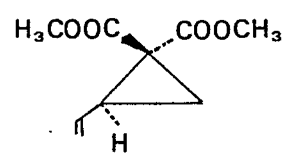

mit Methylmalonsäuredimethylester über das Cyclopentanonderivat der Formel 10

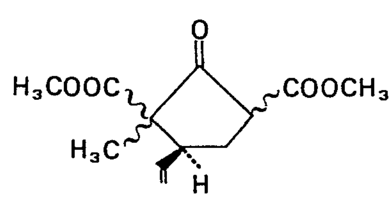

und anschliessende Hydrolyse und Decarboxylierung erhalten wurde, zur Verbindung der Formel 9

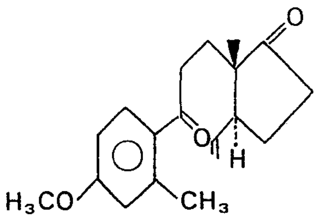

(Ring-AD-Baustein) umsetzt, die Verbindung der Formel 9 in Gegenwart von Kohlenwasserstoffen unter Zusatz von Pyridin/Mesitol mit UV-Licht bei > 340 nm bestrahlt, wobei aus dem dabei entstehenden kurzfristig existenten o-Chinodimethanderivate der Formel 5

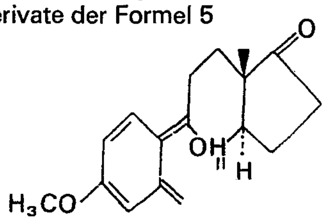

unter den Bestrahlungsbedingungen stereo- und regiospezifisch der 9-α-Hydroxyöstron-3-methyläther der Formel 3

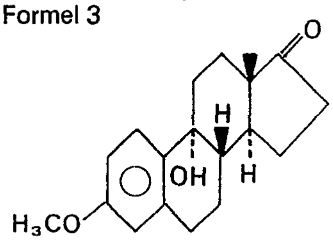

entsteht, der anschliessend durch Dehydratisierung in 9(11)-Dehydroöstron-3-methyläther der Formel 2

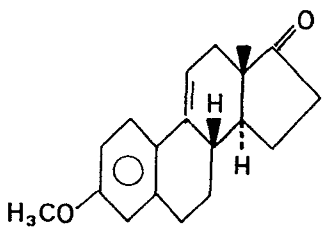

überführt wird und der gegebenenfalls durch katalytische Hydrierung in Östronmethyläther der Formel 1

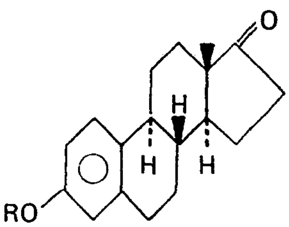

($R=CH_3$) umgewandelt und gegebenenfalls in Östron der Formel 1 (R=H) überführt wird.

Die neue Totalsynthese des Östrons beinhaltet im Falle des Einsatzes von chiralen Ausgangsmaterialien eine asymmetrische bzw. enantiomere Synthese von Östron bzw. Östronderivaten mit überraschend hohen optischen Ausbeuten bei den einzelnen Reaktionsschritten. Sie weist eine Reihe überraschender Reaktionsführungen bzw. Fakten auf:

Es war nicht vorauszusehen, dass durch UV-Bestrahlung des BC-ringoffenen AD-Zwischenbausteins 9 in einer sogenannten optischen Eintopfreaktion *in praxi* in streng stereospezifischer und regiospezifischer Reaktion sogleich das vollständig fertige ABCD-Endprodukt 9-α-Hydroxyöstron-3-methyläther (Formel 3) in guten Ausbeuten (um 60%) als einziges Hauptprodukt gebildet wird. Dieses Faktum steht bisher einzigartig unter sämtlichen bisher bekannten Östrogentotalsynthesen da.

Fernerhin ist der Reaktionsverlauf der für die Synthese des AD-Bausteins 9 erforderlichen Einzelbausteine 11 und 12 c aus den jeweiligen Ausgangsverbindungen 4 bzw. 6 und 7 eigenartig und erfindungswesentlich.

Die Synthese von Östron (1; R=H) erfolgt nach dem Aufbauschema A+D→AD→ABCD. Die nachfolgenden Schemata sowie die Beschreibung sollen die Synthese weiter erläutern.

*1.1. Das o-Chinodimethanderivat 5 reagiert unter intramolekularer 4+2-Cycloaddition zu 3.*

Letzteres geht durch Dehydratisierung und katalytische Hydrierung in 1 ($R=CH_3$) über. Das AD-Produkt 9 fungiert als Schlüsselverbindung und liefert durch Photoenolisierung das kinetisch instabile 5. Sein Aufbau geschieht konvergent aus den leicht zugänglichen Eduktkomponenten 4 bzw. 6 und 7 ($R=CH_3$).

Schema 1

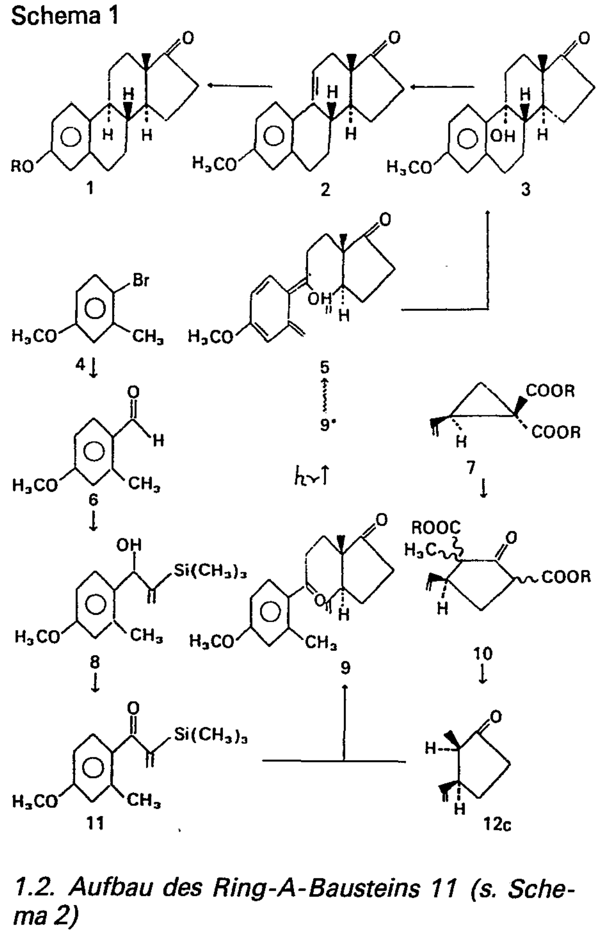


*1.2. Aufbau des Ring-A-Bausteins 11 (s. Schema 2)*

Schema 2 gibt die Darstellung von 11 wieder. Die fetten Pfeile heben den schliesslich bevorzugten Synthesepfad hervor.

Schema 2

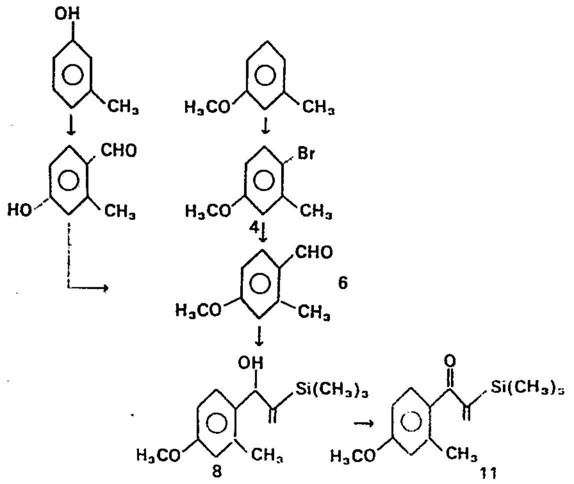


Der Methyläther des m-Kresols (mit 80% durch Einwirkung von Dimethylsulfat auf die wässerigalkalische Lösung aus dem Phenol zugänglich) reagiert bei Temperaturen <0°C mit Brom in $CCl_4$ (in Gegenwart von Eisenpulver) zu 4 (>90%). Die aus 4 *in situ* gewonnene Grignard-Verbindung setzt sich bei Temperaturen <−20°C mit Dimethylformamid in Äther zu 6 (70%) um. Den Aldehyden 6 führt man durch Umsetzung mit der aus 1-Bromvinyltrimethylsilan erhaltenen Grignard-Verbindung bei ca. 50°C in Tetrahydrofuran in 8 (97%) über. Durch Jones-Oxidation entsteht 11 (80%).

*1.3. Aufbau des Ring-D-Bausteins 12 (s. Schema 3)*

Schema 3

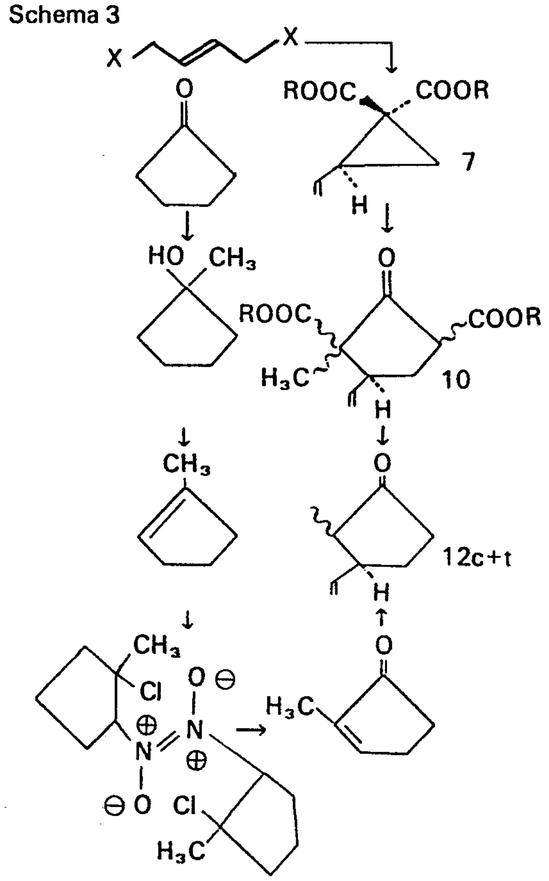


Schema 3 gibt die Bemühungen zur Darstellung von 12 wieder. Die fetten Pfeile heben den bevorzugten Synthesepfad hervor.

Der durch Umsetzung von Malonsäuredimethylester mit (E)-1,4-Dichlorbuten in natriummethylathaltigem Methanol bei 55°C bequem erhältliche 2-Vinylcyclopropan-1,1-dicarbonsäuredimethylester 7 (ca. 60%) liefert bei Reaktion mit Methylmalonsäuredimethylester in natriummethylathaltiger Methanollösung (der Hexamethylphosphorsäuretriamid zugesetzt worden war) das Cyclopentanonderivat 10 (ca. 65%). Das Gemisch von Stereoisomeren ergibt nach Hydrolyse (10 h Kochen unter Rückfluss in wässerig/methanolischer Natronlauge) und Decarboxylierung bei pH5 den Ring-D-Baustein (12c+12t=5:95) (ca. 70%).

Durch Racematspaltung von (RS)−7 (R=H, $CH_3$) mit Brucin ist (R)−7 zugänglich, $[\alpha]_D=+55{,}0$. Eine asymmetrische Synthese von 7 ist wie folgt möglich: Malonsäurediester der Formel

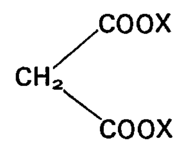

worin O–X einen optisch aktiven Alkoholrest, vorzugsweise einen Rest der Formel

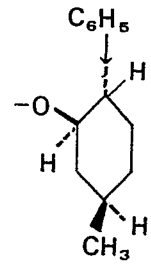

darstellt, wird umgesetzt mit 1,4-Dichlorbuten (=diastereoselektive Cyclopropanierung), anschliessende Hydrolyse und Veresterung mit Diazomethan führt zu optisch aktivem (R)−7, $[\alpha]_D=43{,}9$ (C $Cl_4$).

*1.4. Aufbau und Cyclisierung des AD-Bausteins 9 (s. Schema 1)*

Die Eduktkomponenten 11 und 12 werden mit Na-t-butylat in t-Butanol gelöst und bei Raumtemperatur zu 9 umgesetzt (ca. 60% in bezug auf 11 oder auf 12). Nach 12 h Bestrahlen einer Methylcyclohexanlösung, welche das Michael-Addukt 9, Pyridin (7 mol-Eq) und Mesitol (8 mol-Eq) enthält, bei 95°C mit UV-Licht der Wellenlänge>340 nm gewinnt man ein Reaktionsprodukt, das hauptsächlich aus 3 besteht. Das vom Lösungsmittel befreite Rohprodukt nimmt man in Benzol auf und kocht die Lösung nach Zugabe von Oxalsäure (10 mol-Eq) 20 min unter Rückfluss. Durch präp. HPLC isoliert man ein Dehydratisierungsprodukt (60% bezogen auf 9), das hauptsächlich aus 2 und untergeordnet (ca. 5%) aus 8,9-Dehydroöstronmethyläther besteht.

Die nach dem erfindungsgemässen Verfahren erhältlichen Östrogene bzw. Östrogenderivate finden die gleiche Anwendung wie die auf anderen

Wegen erhältlichen. Beispiele für die Anwendungsgebiete wurden bereits in der Beschreibungseinleitung genannt.

*Beispiel 1:*

*Synthese der racemischen Verbindung*

(4)→(6) (4) (150 mmol) in Äther (150 ml) unter Sieden zu Mg (160 mmol) in Äther (150 ml) getropft und 1 h Rückflusskochen. Anschliessend während 1,5 h bei −40°C $HCON(CH_3)_2$ (458 mmol) in Äther (50 ml) getropft und 2 h gerührt (Temperaturanstieg bis auf −20°C) ergibt (6) (75% nach Dest. i. Vak.).

(6)→(8) 1-Bromvinyltrimethylsilan * (452 mmol) in Tetrahydrofuran (100 ml) bei Temp. < 50°C zu Mg (452 mmol) in THP (200 ml) getropft und 30 min gerührt. Anschliessend zwischen 5-10°C (6) (273 mmol) in Äther(100 ml) gegeben und 12 h gerührt liefert (8) (97% Rohausbeute; analytische Probe durch präp. SC (Benzol/Essigester=4:1) gereinigt).

(8)→(11) Jones-Reagenzlösung ** (100 ml) bei Temp. < 10°C zu (8) (268 mmol) in Äther (1 l) getropft und 15 h bei Raumtemp. gerührt führt zu (11; $R=CH_3$) (80% nach Dest. i. Vak.).

(7)→(10) Methylmalonsäuredimethylester (205 mmol) in Methanol (20 ml) in eine Lösung von $NaOCH_3$(217,5 mmol) in Methanol (125 ml) getropft. Nach 10-min-Rühren bei 50°C Zugabe von (7; $R=CH_3$) (200 mmol) in Hexamethylphosphorsäuretriamid (25 ml) und Methanol (20 ml). Während 1,5 h scharfes Einengen (Badtemp. 100-110°C). Nach 8 h Verteilen des Reaktionsgutes zwischen Äther (250 ml) und 10%wässerig. $H_2SO_4$ (250 ml) erhält man (10; $R=CH_3$) 67% (nach Dest. i. Vak.; Drehbandkolonne).

(10;$R=CH_3$)→(12) (10; $R=CH_3$) (72,4 mmol) in Methanol (50 ml) zu NaOH (260 mmol) in Wasser (250 ml) gegeben, 12 h bei Raumtemp. gehalten und 10 h Rückfluss kochen. Mit konz. Salzsäure neutralisiert (Bromthymolblau) und während 2 h mit $NaH_2PO_4 \cdot 2H_2O$ versetzt. Nach 4 h mit Pentan extrahiert und Dest. i. Kugelrohr gibt (12c) und (12t) (76%)=5:95 (lt. GC).

(11)+(12c+t)→(9) Zu (11) (20,0 mmol) und (12c+t) (30,2 mmol) in Äther (200 ml) tropfte man während 1 h unter Rühren bei Raumtemp. 0,1 n $NaOC(CH_3)_3$ in $HOC(CH_3)_3$ (8 ml). Nach 12 h in 10% wässerig. $H_2SO_4$ (200 ml) und Methanol (50 ml) eingetragen und 48 h gerührt. Nach Kugelrohrdest. i. Vak., präp. HPLC (Petroläther/Essigester=10+2; 2 Silicakartuschen Waters, 0,15 l/min; 1 mal Recycl.) und Kugelrohrdest. isolierte man (9) (12,9 mmol) (64,5% bezogen auf [11]).

(9)→(2) (9) (1,72 mmol), Mesitol (14,7 mmol), Pyridin (12,5 mmol) in Methylcyclohexan (450 ml) bei 98°C im Rayonet-Reactor (3500 Å-Lampensatz; Wellenlängenbereich <340 nm weggefiltert) 12 h unter Durchleiten von $N_2$ bestrahlt. Nach Aufnehmen des getrockneten Bestrahlungsrohprodukts in Benzol (300 ml) und Zugabe von Oxalsäure (16,7 mmol) 20 min Rückflusskochen liefert (2) [54% bezogen auf (9)] nach präp. HPLC (Petroläther/Essigester =10+2:2 Silicakartuschen Waters, 0,1 l/min; 2mal Recycl.).

* A. Ottolenghi, M. Fridkin und A. Zilkha, „Canad. J. Chem.", *41*, 2977 (1963).

** K. Bowden, I.M. Heilbronn, E.R.H. Jones und B.C.L. Weedon, „J. Chem. Soc.," *1946*, 39.

*Charakterisierung der Verbindungen*

(1; $R=CH_3$)

Fp. 142-144°C (aus Methanol). UV (Methanol); $\lambda_{max}(\varepsilon)$ 276 (1900), 286 nm (2000). IR (KBr): 1735 (s, 5-Ringketon, 1610, 1580, 1505 $cm^{-1}$ (m, w bzw. m, Benzolring). $^1$H-NMR ($CDCl_3$): δ=0,90 (s, 3H, $CH_3$ an C), 1,23-3,10 (m, 15H, Methylen- und Methinprotonen am Steroidgerüst), 3,77 (s, 3H, $CH_3$ an O), 6,58-6,83 sowie 7,07-7,30 (jeweils m; 2H bzw. 1H vom Ring A). $^{13}$C-NMR ($CDCl_3$)δ=13,78 (q, C-18), 21,51 (t, C-15), 25,93 (t, C-11), 26,52 (t, C-7), 29,64 (t, C-6), 31,59 (t, C-12), 35,74 (t, C-16), 38,34 (d, C-8), 43,93 (d, C-9), 47,90 (s, C-13), 50,37 (d, C-14), 55,05 (q, $OCH_3$), 111,52 (d, C-2), 113,86 (d, C-4), 126,21 (d, C-1), 131,93 (s, C-10), 137,58 (s, C-5), 157,60 (s, C-3), 220,38 (s, C-17).

(2)

Fp. 150-152°C (aus Methanol). UV (Methanol): $\lambda_{max.}(\varepsilon)$=262 (19400), Schulter bei 296 nm (3350). IR (KBr): 3020 (w, C=C-H), 1740 (s,α-Ringketon), 1620 (w, C=C), 1610, 1575, 1500 $cm^{-1}$ (jeweils m, Benzolring). $^1$H-NMR ($CDCl_3$): δ=0,93 (s, 3H, $CH_3$ an C), 1,23-307 (m, 12H, Methylen- und Methinprotonen am Steroidgerüst), 3,78 (s, 3H, $CH_3$ an O), 6,13 (m, 1H, 11H, 6,55-6,86 sowie 7,41-7,65 (jeweils m, 1, 2 bzw. 4H). $^{13}$C-NMR ($CDCl_3$): δ=14,34 (q, C-18), 22,35 (t, C-15), 27,66 (t, C-7), 29,77 (t, C-6), 33,84 (t, C-12), 36,02 (t, C-16), 38,10 (d, C-8), 46,03 (s, C-13), 47,70 (d, C-14), 55,04 (q, $OCH_3$) 112,65 (d, C-2), 113,27 (d, C-4), 116,53 (d, C-11), 125,22 (d, C-1), 127,00 (s, C-9), 135,30 (s, C-10), 137,30 (s, C-5), 158,53 (s, C-3), 221,21 (s, C-17).

(3)

Fp. 172-174°C (aus Methylenchlorid/Äther; ab 140°C langsam beginnende Zersetzung). UV (Methanol): $\lambda_{max.}(\varepsilon)$=220 (9100), 274 (1690), 282 nm (1635). IR (KBr): 3500 (s, OH), 1725 (5-Ringketon), 1610, 1580, 1500 cm (s, m bzw. s; Benzolring). $^1$H-NMR ($CDCl_3$): δ=0,90 (s, 3H, $CH_3$ an C-13), 1,47 (s, 1H, OH), 1,60 (m, 1H), 1,80 (m, 6H), 1,96 (m, $J_{15,15}$ 12 Hz, H-15α), 2,13 (m, 2H), 2,48 (m, 2H), 2,90 (m, 2H, H-6), 3,79 (s, 3H, $CH_3$ an O), 6,66 (d, $J_{2,4}$=2,5 Hz, 1H, H-4), 6,76 (dd, $J_{1,2}$=8,7, $J_{2,4}$=2,7 Hz, 1H, H-2), 7,45 (d, $J_{1,2}$=8,9 Hz, 1H H-1). $^{13}$C-NMR ($CDCl_3$): δ=12,93 (q, C-18), 20,21 (t, C-7), 21,47 (t, C-15), 27,75 (t, C-12), 29,70 (t, C-6), 32,30 (t, C-11), 35,94 (t, C-16), 41,46 (d, C-8), 43,15 (d, C-14), 47,70 (s, C-13), 55,24 (q, $CH_3$ an O), 69,99 (s, C-9), 112,37 (d, C-2), 114,06 (d, C-4), 126,47 (d, C-1), 134,27 (s, C-10), 138,23 (s, C-5), 158,97 (s, C-3), 220,51 (s, C-17).

(8)

UV (Cyclohexan): $\lambda_{max.}(\varepsilon)$=233 (8600), 278 (1340), 284 nm (1290). IR (Film): 3200-3600 (s, OH), 1615 (s, CC-Doppelbindung), 1585 und 1505 (m bzw. s, Benzolring), 840 (s, Si-$CH_3$), 810 $cm^{-1}$ (Vinylidengruppierung). $^1$H-NMR ($CDCl_3$; Standard: Cyclohexan): −0,04 (s, 9H, $CH_3$ an Si), 1,7 (m, 1H, OH), 2,24 (s, 3H, $CH_3$ an C), 3,80 (s, 3H, $CH_3$ an O), 5,50 (m, 1H, allyl. H), 5,59 (m, 1H, olefin. H, cis zu Si-Ligand), 5,84 (m, 1H, olefin. H, trans zu Si-Ligand), 6,73 (m, 2H, H-2 und H-4), 7,25 (m, 1H, H-1).

(9)

Kp. 190°C 0,2 Torr. UV (Cyclohexan): $\lambda_{max.}(\varepsilon)$=265 nm (16400), Schultern bei 325 (200), 350 nm (75). IR (Film): 1738 (s, 5-Ringketon), 1677 (s, konj.-unges. Keton), 1640 (w, CC-Doppelbindung), 1605 und 1570 $cm^{-1}$ (Benzolring). $^1$H-NMR ($CDCl_3$): δ=0,91 (s, 3H, $CH_3$ an C-13), 1,71-3,1 (m, 9H, aliphat. H), 2,53 (s, 3H, $CH_3$ an C-5), 3,84 (s, 3H, $CH_3$ an O), 5,12-5,19 (m, 2H, 2 olefin. H), 5,76-5,89 (m, 1H, 1 olefin. H), 6,76 (m, 2H, aromat. H), 7,73 (m, 1H, aromat. H). $^{13}$C-NMR ($CDCl_3$): δ=17,4 (q, C-18), 22,36 (q, C-6), 24,31 (t, C-12), 30,54 (t, C-15), 35,48 (t, C-11/C-16), 36,78 (t, C-16/C-11), 48,48 (d, C-14), 50,82 (s, C-13), 55,11 (q, $CH_3$ an O), 110,48 (d, C-2), 116,59 (t, C-7), 117,50 (d, C-4), 129,85 (s, C-10), 131,67 (d, C-1), 137,13 (d, C-8), 141,67 (s, C-5), 161,69 (s, C-3), 200,95 (s, C-9), 221,36 (s, C-17).

(11)

Kp. 105°C 0,2 Torr. UV (Cyclohexan): $\lambda_{max.}(\varepsilon)$=275 nm (8900), IR (Film): 1648 (s, konj.-unges. Keton), 1610 und 1520 (s, bzw. m, Benzolring), 8,40 (s, Si-$CH_3$) 800 $cm^{-1}$ (Vinylidengruppierung). $^1$H-NMR ($CDCl_3$): δ=0,21 (s, 9H, $CH_3$ an Si), 2,45 (s, 3H, $CH_3$ an C), 3,83 (s, 3H, $CH_3$ an O) 6,06 (q, AB-System, 2 olefin. H), 6,71 (dd, J=2,6 Hz, J=8,5 Hz, 1H, H-2), 6,76 (d, J=2,6 Hz, 1H, H-4), 7,39 (d, J=8,5 Hz, 1H, H-1).

(12 c)

IR (Film): 3080 (m, C=$CH_2$), 1740 (s, 5-Ringketon), 1645 und 915 $cm^{-1}$ (C=$CH_2$). $^1$H-NMR ($CDCl_3$): δ=0,98 (d, J=7,6 Hz, 3H, $CH_3$), 1,85-2,00 (m, 1H, aliphat. H), 2,00-2,15 (m, 1H, aliphat. H), 2,23-2,31 (m, 2H, aliphat. H), 2,23-2,31 (m, 2H, aliphat. H), 2,36 (o zum q vereinfacht, 1H, H-13; nach Sättigen des Signals bei 0,98 ppm d, $J_{13,14}$=7,8 Hz), 2,96 (m, aliphat. H), 5,04-5,17 (m, 2H, olefin. H), 5,61-5,78 (m, 1H, olefin. H).

(12 t)

IR (Film): 3080 (m, C=$CH_2$), 1740 (s, 5-Ringketon), 1645 u. 915 $cm^{-1}$ (C=$CH_2$). $^1$H-NMR ($CDCl_3$): δ=1,05 (d, J=6,8 Hz, 3H, $CH_3$), 1,58-1,74 (m, 1H, aliphat. H), 1,87 (o als 6-Linien-m sichtbar, 1H, H-13; nach Sättigen des Signals bei 1,05 ppm d, $J_{13,14}$=11,7 Hz), 2,04-2,44 (m, 4H, aliphat. H), 5,04-5,20 (m, 2H, olefin. H), 5,73-5,89 (m, 1H, olefin. H).

*Beispiel 2:*

*Synthese der (+) Verbindung der Formel 2 (R=$CH_3$)*

(4)→(6) (4) (150 mmol) in Äther (150 ml) unter Sieden zu Mg (160 mmol) in Äther (150 ml) getropft und 1 h Rückflusskochen. Anschliessend während 1,5 h bei −40°C $HCON(CH_3)_2$ (458 mmol) in Äther (50 ml) getropft und 2 h gerührt (Temperaturanstieg bis auf −20°C) ergibt (6) (75% nach Dest. i. Vak.).

(6)→(8) 1-Bromvinyltrimethylsilan * (452 mmol) in Tetrahydrofuran (100 ml) bei Temp. <50°C zu Mg (452 mmol) in THP (200 ml) getropft und 30 min gerührt. Anschliessend zwischen 5-10°C (6) (273 mmol) in Äther (100 ml) gegeben und 12 h gerührt liefert (8) (97% Rohausbeute; analytische Probe durch präp. SC [Benzol/Essigester=4:1]) gereinigt.

(8)→(11) Jones-Reagenzlösung ** (100 ml) bei Temp. <10°C zu (8) (268 mmol) in Äther (1 l) getropft und 15 h bei Raumtemperatur gerührt führt zu (11; R=$CH_3$) (80% nach Dest. i. Vak.).

(R)-(7)→opt. akt.(10) Methylmalonsäuredimethylester (205 mmol) in Methanol (20 ml) in eine Lösung von $NaOCH_3$ (217,5 mmol) in Methanol (125 ml) getropft. Nach 10 min. Rühren bei 50°C Zugabe von (R)-(7) (R=$CH_3$, $[\delta]_D$+55°C) (200 mmol) in Hexamethylphosphorsäuretriamid (25 ml) und Methanol (20 ml). Während 1,5 h scharfes Einengen (Badtemp. 100-110°C). Nach 8 h Verteilen des Reaktionsgutes zwischen Äther (250 ml) und 10% wässerig. $H_2SO_4$ (250 ml) erhält man opt. akt. (10); (R=$CH_3$) (67% nach Dest. i. Vak. Drehbandkolonne).

Opt. akt.(10) (R=$CH_3$)→(+)−(12)c Opt. akt. (10) (R=$CH_3$) (72,4 mmol) in Methanol (50 ml) zu NaOH (260 mmol) in Wasser (250 ml) gegeben, 12 h bei Raumtemperatur gehalten und 10 h Rückflusskochen. Mit konzentrierter Salzsäure neutralisiert (Bromthymolblau) und während 2 h mit $NaH_2PO_4 \cdot 2H_2O$ versetzt. Nach 4 h mit Pentan extrahiert und Dest. im Kugelrohr gibt (+)- (12)c, $[\alpha]_D$+149° ($CH_2Cl$) nach Umkristallisation.

(11)+ (+)− (12)c→(+)−(9) Zu (11) (20,0 mmol) und (12)c (30,2 mmol) in Äther (200 ml) tropfte man während 1 h unter Rühren bei Raumtemperatur 0,1N $NaOC(CH_3)_3$ in $HOC(CH_3)_3$ (8 ml). Nach 12 h in 10% wässerig. $H_2SO_4$ (200 ml) und Methanol (50 ml) eingetragen und 48 h gerührt. Nach Kugelrohrdestillation im Vakuum, präp. HPLC (Petroläther/Essigester=10+2; 2 Silicakartuschen Waters, 0,15 l/min; 1mal Recycl.) und Kugelrohrdestillation isolierte man (+) (9) (12,9 mmol) (64,5% bezogen auf [11]), $[\alpha]_D$=27,1° ($CHCl_3$).

(+)−(9)→(+)−(2) (+)−(9) (1,72 mmol), Mesitol (14,7 mmol), Pyridin (12,5 mmol) in Methylcyclohexan (450 ml) bei 98°C im Rayonet-Reactor (3500 Å-Lampensatz; Wellenlängenbereich <340 nm weggefiltert) 12 h unter Durchleiten von $N_2$ bestrahlt. Nach Aufnehmen des getrockneten Bestrahcungsrohprodukts in Benzol (300 ml) und Zugabe von Oxalsäure (16,7 mmol) 20 min. Rückflusskochen liefert (+)−(2) (54%

bezogen auf (+)−(9)) nach präp. HPLC (Petroläther/Essigester=10+2; 2 Silicakartuschen Waters, 0,1 l/min; 2mal Recycl.), $[\alpha]_D$+288,6° (Dioxan).

* A. Ottolenghi, M. Fridkin und A. Zilkha, „Canad. J. Chem.", *41*, 2977 (1963).

** K. Bowden, I.M. Heilbronn, E.R.H. Jones und B.C.L. Weedon, „J. Chem. Soc.", *1946*, 39.

*Beispiel 3:*

*Synthese der (+)-Verbindung der Formel 2 (R=$CH_3$)*

(4)→(6) (4) (150 mmol) in Äther (150 ml) unter Sieden zu Mg (160 mmol) in Äther (150 ml) getropft und 1 h Rückflusskochen. Anschliessend während 1,5 h bei −40°C $HCON(CH_3)_2$ (458 mmol) in Äther (50 ml) getropft und 2 h gerührt (Temperaturanstieg bis auf −20°C) ergibt (6) (75% nach Dest. i. Vak.).

(6)→(8) 1-Bromvinyltrimethylsilan * (452 mmol) in Tetrahydrofuran (100 ml) bei Temp. < 50°C zu Mg (452 mmol) in THP (200 ml) getropft und 30 min gerührt. Anschliessend zwischen 5-10°C (6) (273 mmol) in Äther (100 ml) gegeben und 12 h gerührt liefert (8) (97% Rohausbeute; analytische Probe durch präp. SC [Benzol/Essigester=4:1]) gereinigt.

(8)→(11) Jones-Reagenzlösung ** (100 ml) bei Temp. < 10°C zu (8) (268 mmol) in Äther (1 l) getropft und 15 h bei Raumtemp. gerührt führt zu (11; R=$CH_3$) (80% nach Dest. i. Vak.).

(R)-(7)→opt. akt. (10) Methylmalonsäuremethylester (205 mmol) in Methanol (20 ml) in eine Lösung von $NaOCH_3$ (217,5 mmol) in Methanol (125 ml) getropft. Nach 10 min Rühren bei 50°C Zugabe von (R)-(7), $[\alpha]_D$=+43,9° (R=$CH_3$) (200 mmol) in Hexamethylphosphorsäuretriamid (25 ml) und Methanol (20 ml). Während 1,5 h scharfes Einengen (Badtemp. 100-110°C). Nach 8 h Verteilen des Reaktionsgutes zwischen Äther (250 ml) und 10 proz. wässr. $H_2SO_4$ (250 ml) erhält man opt. akt. (10) (R=$CH_3$) 67% (nach Dest. i. Vak.; Drehbandkolonne).

Opt. akt. (10) (R=$CH_3$)→(+)−(12)c Opt. akt. (10) (R=$CH_3$) (72,4 mmol) in Methanol (50 ml) zu NaOH (260 mmol) in Wasser (250 ml) gegeben, 12 h bei Raumtemperatur gehalten und 10 h Rückflusskochen. Mit konz. Salzsäure neutralisiert (Bromthymolblau) und während 2 h mit $NaH_2PO_4 \cdot 2H_2O$ versetzt. Nach 4 h mit Pentan extrahiert und Dest. i. Kügelrohr gibt (+)−(12)c $[\alpha]_D$=112,7, opt. Ausbeute 76%.

(11)+(+)−(12)c→(+)−(9) Zu (11) (20,0 mmol) und (12)c+t (30,2 mmol) in Äther (200 ml) tropfte man während 1 h unter Rühren bei Raumtemperatur 0,1N $NaOC(CH_3)_3$ in $HOC(CH_3)_3$ (8 ml). Nach 12 h in 10%wässerig $H_2SO_4$ (200 ml) und Methanol (50 ml) eingetragen und 48 h gerührt. Nach Kugelrohrdestillation im Vakuum, präp. HPLC (Petroläther/Essigester=10+2; 2 Silicakartuschen Waters, 0,15 l/min; 1mal Recycl.) und Kugelrohrdestillation isolierte man (+)−(9) (12,9 mmol) (64,5% bezogen auf (11), $[\alpha]_D$=21,2, opt. Ausbeute: 80%.

(+)−(9)→(+)−(2) (+)−(9) (1,72 mmol), Mesitol (14,7 mmol), Pyridin (12,5 mmol) in Methylcyclohexan (450 ml) bei 98°C im Rayonet-Reactor (3500 Å-Lampensatz; Wellenlängenbereich < 340 nm weggefiltert) 12 h unter Durchleiten von $N_2$ bestrahlt. Nach Aufnehmen des getrockneten Bestrahlungsrohprodukts in Benzol (300 ml) und Zugabe von Oxalsäure (16,7 mmol) 20 min. Rückflusskochen liefert (+)−(2) (54% bezogen auf (+)−(9)) nach präp. HPLC (Petroläther/Essigester=10+2; 2 Silicakartuschen Waters, 0,1 l/min; 2mal Recycl.), $[\alpha]_D$=Y+231,8, opt. Ausbeute: 80%.

* A. Ottolenghi, M. Fridkin und A. Zilkha, „Canad. J. Chem.", *41*, 2977 (1963).

** K. Bowden, I.M. Heilbronn, E.R.H. Jones und B.C.L. Weedon, „J. Chem. Soc.", *1946*, 39.

Die Ausgangsverbindung (R)-(7), $[\alpha]_D$=+43,9° wird, wie beschrieben, erhalten, die opt. Ausbeute beträgt 80%, d.h. es sind noch Verunreinigungen an (S)-(7) anwesend, die im weiteren Verlauf nicht aufgeführt wurden.

**Patentansprüche**

1. Verfahren zur Synthese von Östron oder Östronderivaten, dadurch gekennzeichnet, dass man den Aldehyd der Formel 6

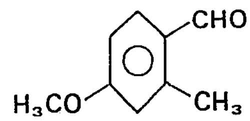

mit der Grignard-Verbindung aus 1-Bromvinyltrimethylsilan zur Verbindung der Formel 8

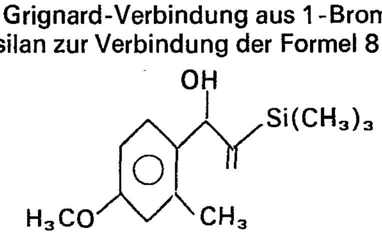

umsetzt, diese zur Verbindung der Formel 11

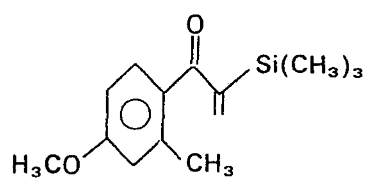

oxidiert (Ring-A-Baustein), diese durch mit Alkali- und Erdalkalialkoholaten katalysierte Michael-Reaktion mit der Verbindung der Formel 12 c

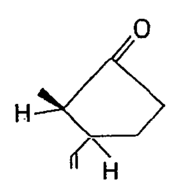

(Ring-D-Baustein), welche durch Reaktion von 2-Vinylcyclopropan-1,1-dicarbonsäuredimethylester der Formel 7

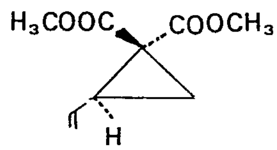

mit Methylmalonsäuredimethylester über das Cyclopentanonderivat der Formel 10

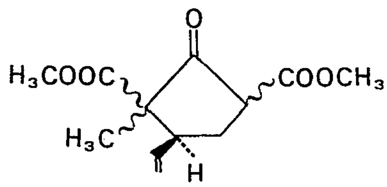

und anschliessende Hydrolyse und Decarboxylierung erhalten wurde, zur Verbindung der Formel 9

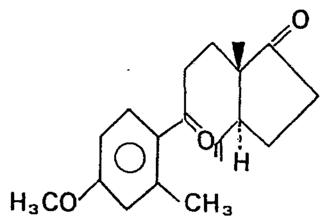

(Ring-AD-Baustein) umsetzt, die Verbindung der Formel 9 in Gegenwart von Kohlenwasserstoffen unter Zusatz von Pyridin/Mesitol mit UV-Licht bei >340 nm bestrahlt, wobei aus dem anbei entstehenden kurzfristig existenten o-Chinodimethanderivate der Formel 5

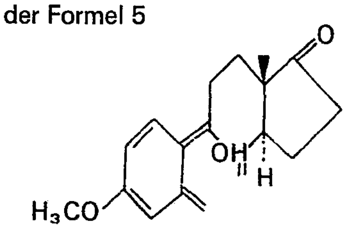

unter den Bestrahlungsbedingungen stereo- und regiospezifisch der 9-α-Hydroxyöstron-3-methyläther der Formel 3

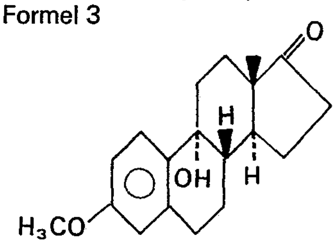

entsteht, der anschliessend durch übliche Dehydratisierung in 9(11)-Dehydroöstron-3-methyläther der Formel 2

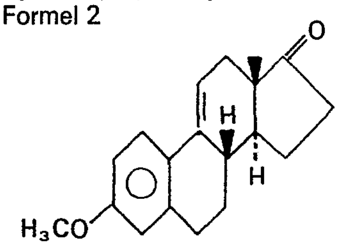

überführt wird und der gegebenenfalls durch katalytische Hydrierung in Östronmethyläther der Formel 1

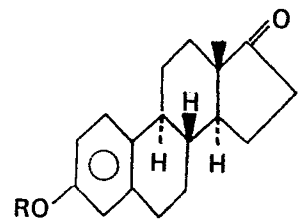

($R=CH_3$) umgewandelt und gegebenenfalls in Östron der Formel 1 (R=H) überführt wird.

2. 1-(4-Methoxy-2-methylphenyl)-3-(1-methyl-2-oxo-5t-vinylcyclopent-r-yl)propan-1-on der Formel 9 gemäss Anspruch 1.

## Claims

1. Process for synthesizing estrone or estrone derivatives, characterized by reacting the aldehyde of the formula 6

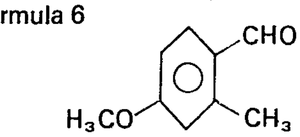

with the Grignard compound formed from 1-bromovinyltrimethylsilane to give the compound of the formula 8

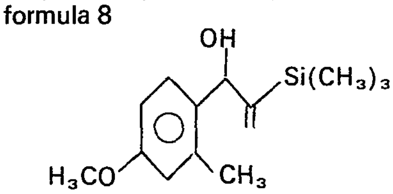

oxidizing the latter to give the compound of the formula 11

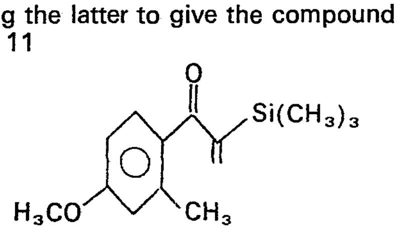

(the ring A structural unit), reacting the latter, by a Michael reaction catalyzed by alkali metal and alkaline earth metal alcoholates, with the compound of the formula 12 c

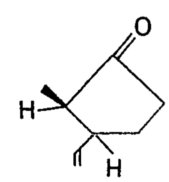

(the ring D structural unit), which has been obtained by reacting 2-vinylcyclopropane-1,1-dicarboxylic acid dimethyl ester of the formula 7

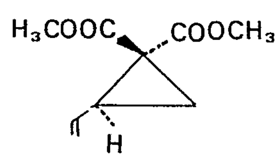

with methylmalonic acid dimethyl ester, *via* the cyclopentanone derivative of the formula 10

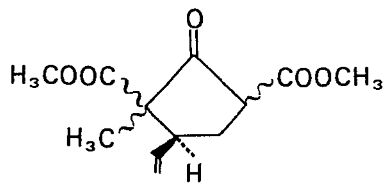

and subsequent hydrolysis and decarboxylation, to give the compound of the formula 9

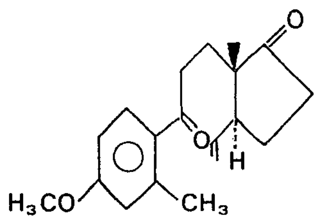

(the ring AD structural unit), and irradiating the compound of the formula 9 with UV light of a wavelength > 340 nm in the presence of hydrocarbons and with the addition of pyridine/mesitol, whereupon the short-life o-quinodimethane derivatives thus formed, of the formula 5

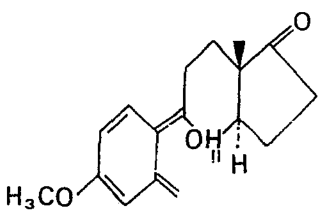

under the conditions of irradiation gives, in a stereo-specific and regio-specific reaction, the 9-α-hydroxyestrone 3-methyl ether of the formula 3

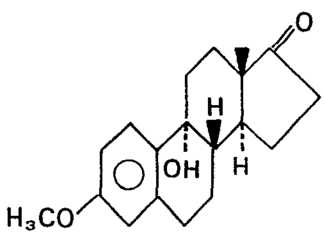

which is then converted by dehydration into 9(11)-dehydroestrone 3-methyl ether of the formula 2

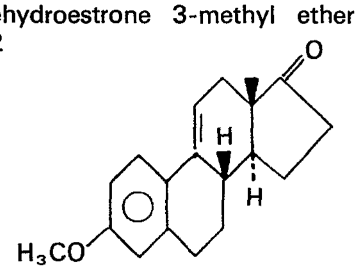

which, if desired, is converted by catalytic hydrogenation into estrone methyl ether of the formula 1

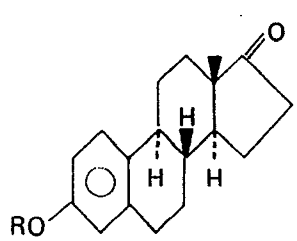

($R=CH_3$) and, if desired, is converted into estrone of the formula 1 ($R=H$).

2. 1-(4-Methoxy-2-methylphenyl)-3-(1-methyl-2-oxo-5t-vinylcyclopent-r-yl)propan-1-one of the formula 9 as claimed in Claim 1.

**Revendications**

1. Procédé de synthèse de l'œstrone ou de ses dérivés, caractérisé en ce qu'on fait réagir l'aldéhyde de formule 6

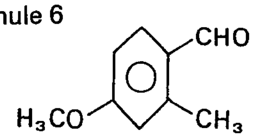

avec le composé de Grignard du 1-bromovinyltriméthylsilane, pour obtenir le composé de formule 8

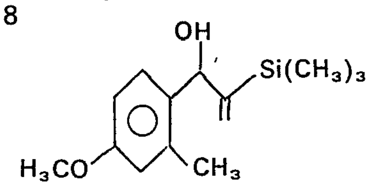

en ce qu'on oxyde ce composé en composé de formule 11 (squelette cyclique A)

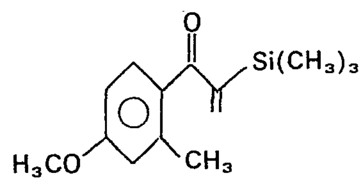

en ce qu'on fait réagir celui-ci par une réaction de Michael catalysée avec des alcoolates alcalins et alcalino-terreux, avec le composé de formule 12 c

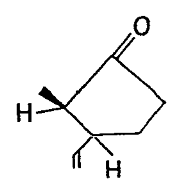

(squelette cyclique D), en ce qu'on obtient par réaction de l'ester diméthylique de l'acide 2-vinylcyclopropane-1,1-dicarboxylique de formule 7

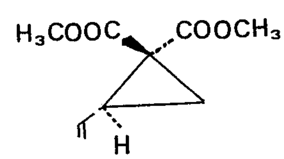

avec l'ester diméthylique de l'acide méthylmalonique en passant par le dérivé de cyclopentanone de formule 10

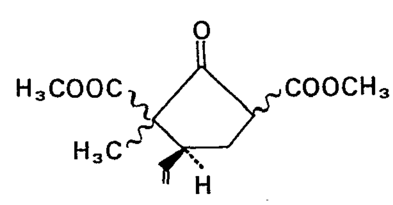

et en ce qu'on hydrolyse et décarboxylise pour aboutir au composé de formule 9

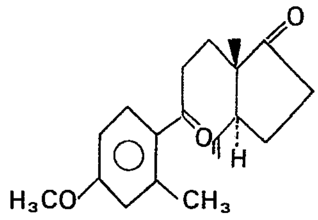

(squelette cyclique AD), en ce qu'on irradie le composé de formule 9 à la lumière ultraviolette à plus de 340 nm en présence d'hydrocarbures et en ajoutant un mélange de pyridine/mésitol, auquel cas à partir du dérivé d'o-quinonediméthane existant rapidement — ainsi formé — de formule 5

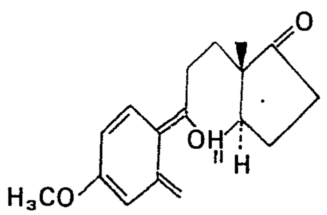

dans les conditions d'irradiation, il se forme stéréo- et régio-spécifiquement l'éther 3-méthylique de la 9-α-hydroxyœstrone de formule 3

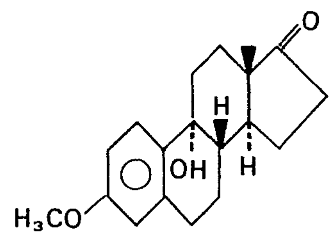

en ce qu'on convertit finalement par déshydratation en l'éther-3 méthylique de la 9(11)-déshydro-œstrone de formule 2

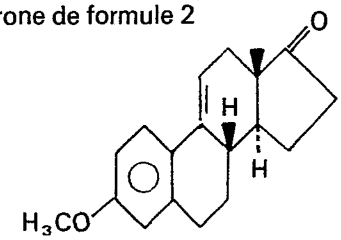

et en ce qu'on transforme éventuellement par hydrogénation catalytique en l'éther méthylique d'œstrone de formule 1

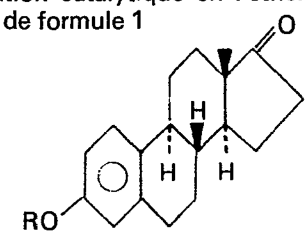

($R=CH_3$) et en ce qu'on convertit éventuellement en œstrone de formule 1 ($R=H$).

2. 1-(4-méthoxy-2-méthylphényl)-3-(1-méthyl-2-oxo-5t-vinylcyclopent-r-yl)propane-1-one de formule 9 selon la revendication 1.